(19) Europäisches Patentamt
European Patent Office
Office européen des brevets

(11) Veröffentlichungsnummer: **0 438 554 B1**

(12) # EUROPÄISCHE PATENTSCHRIFT

(45) Veröffentlichungstag der Patentschrift: **08.06.94**

(51) Int. Cl.$^5$: **C07D 309/22**, A61K 31/35, A01N 43/16

(21) Anmeldenummer: **90910646.0**

(22) Anmeldetag: **05.07.90**

(86) Internationale Anmeldenummer: **PCT/EP90/01082**

(87) Internationale Veröffentlichungsnummer: **WO 91/00860 (24.01.91 91/03)**

(54) **STICKSTOFFHALTIGE AMBRUTICINE, VERFAHREN ZU IHRER HERSTELLUNG UND IHRE VERWENDUNG.**

(30) Priorität: **06.07.89 DE 3922283**

(43) Veröffentlichungstag der Anmeldung: **31.07.91 Patentblatt 91/31**

(45) Bekanntmachung des Hinweises auf die Patenterteilung: **08.06.94 Patentblatt 94/23**

(84) Benannte Vertragsstaaten: **AT BE CH DE DK ES FR GB IT LI LU NL SE**

(56) Entgegenhaltungen:

**J. Med Chem., Band 22, Nr. 9, 1979, D.T. Connor et al; Seite 1055-59 siehe Verbindung 29**

**J. Med. Chem., Band 22, Nr. 9, 1979, D.T. Connor et al; Seite 1144-47, siehe Verbindung 16 und 17**

(73) Patentinhaber: **Gesellschaft für Biotechnologische Forschung mbH (GBF)
Mascheroder Weg 1
D-38124 Braunschweig(DE)**

(72) Erfinder: **BEDORF, Norbert
Mascheroder Weg 1
D-3300 Braunschweig(DE)**
Erfinder: **FORCHE, Edgar
Mascheroder Weg 1
D-3300 Braunschweig(DE)**
Erfinder: **GERTH, Klaus
Mascheroder Weg 1
D-3300 Braunschweig(DE)**
Erfinder: **HÖFLE, Gerhard
Mascheroder Weg 1
D-3300 Braunschweig(DE)**
Erfinder: **IRSCHIK, Herbert
Mascheroder Weg 1
D-3300 Braunschweig(DE)**
Erfinder: **JANSEN, Rolf
Mascheroder Weg 1
D-3300 Braunschweig(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

Erfinder: **KUNZE, Brigitte**
**Mascheroder Weg 1**
**D-3300 Braunschweig(DE)**
Erfinder: **REICHENBACH, Hans**
**Mascheroder Weg 1**
**D-3300 Braunschweig(DE)**
Erfinder: **SASSE, Florenz**
**Mascheroder Weg 1**
**D-3300 Braunschweig(DE)**
Erfinder: **STEINMETZ, Heinrich**
**Mascheroder Weg 1**
**D-3300 Braunschweig(DE)**
Erfinder: **TROWITZSCH-KIENAST, Wolfram**
**Mascheroder Weg 1**
**D-3300 Braunschweig(DE)**

(74) Vertreter: **Boeters, Hans Dietrich, Dr. et al**
**Patentanwälte Boeters Bauer Koepe**
**Bereiteranger 15**
**D-81541 München (DE)**

## Beschreibung

Die vorliegende Erfindung bezieht sich auf neue, stickstoffhaltige Ambruticine, auf ihre Herstellung aus Sorangium (Polyangium) cellulosum So ce 10 (DSM 5386) und auf ihre Verwendung zur Bekämpfung von Pilzinfektionen bei Mensch und Tier bzw. als Pflanzenschutzmittel. Der Vollständigkeit halber sei bemerkt, daß sich die erfindungsgemäßen Ambruticine auch aus anderen Mikroorganismen gewinnen lassen.

Etwa im Jahre 1970 wurde aus einem Myxobakterium, Polyangium cellulosum var. fulvum, eine stickstoffreie Cyclopropyl-polyenpyransäure isoliert (US-PS-3.651.216, Warner-Lambert), die den Namen Ambruticin erhielt und in vitro fungistatische sowie schwach antibakterielle Wirkung zeigt (S.M. Ringel et al., J. of Antibiotics 30, 371 (1977)).

Es ist von großem allgemeinem Interesse, neue fungizide oder fungistatische Substanzen zu finden, deren Wirkungsspektrum sich von dem bekannter Verbindungen unterscheidet. Solche Substanzen vergrößern die Palette an Medikamenten, die der modernen Medizin bei der Bekämpfung der verschiedensten Pilzinfektionen zur Verfügung stehen und vermindern dadurch auch die Gefahr der Resistenzbildung. Auch für den Einsatz in Pflanzenschutzmitteln sind sie wegen ihrer andersartigen Hemmeffekte interessant.

Aus einer Bodenprobe aus Mexiko konnte nun ein Stamm aus der Ordnung der Myxobakterales, Sorangium (Polyangium) cellulosum So ce 10 (DSM 5386) isoliert werden, der neue Verbindungen aus der Gruppe der Ambruticine produziert. Diese zeigen sehr gute in vitro Wirkung gegen humanpathogene sowie phytopathogene Pilze, z. B. gegen Hyphenpilze einschließlich Hautpilze und Aspergillus niger sowie einige Hefen, während sie antibakteriell unwirksam sind. Es gibt auch erste Hinweise auf eine insektizide Wirkung.

Dementsprechend stellt die vorliegende Erfindung stickstoffhaltige Ambruticine und deren pharmazeutisch und agrikulturchemisch annehmbare Salze zur Verfügung, die dadurch gekennzeichnet sind, daß man

(a) Sorangium (Polyangium) cellulosum So ce 10 (DSM 5386) in einem Kohlenstoff- und Stickstoffquellen sowie anorganische Salze enthaltenden Medium in Gegenwart eines Adsorberharzes während der logarithmischen bis zur stationären Wachstumsphase aerob kultiviert,
(b) danach das Adsorberharz von der Kulturbrühe abtrennt und mit Wasser wäscht,
(c) die am Adsorberharz adsorbierten Ambruticine mit einem $C_{1-3}$-Alkanol auswäscht,
(d) das Eluat einengt und lyophilisiert, (e) den Rückstand in einem $C_{1-2}$-Alkanol löst,
(f) die Lösung filtriert und über Sephadex chromatographiert und
(g) die ambruticinhaltigen Fraktionen einengt

und fakultativ die so gewonnenen Ambruticine

(h) chromatographisch trennt
und/oder
(i) in ihre pharmazeutisch annehmbaren Salze überführt.

Des weiteren stellt die Erfindung ein Verfahren zur Herstellung dieser Verbindungen zur Verfügung, das dadurch gekennzeichnet ist, daß man

(a) Sorangium (Polyangium) cellulosum So ce 10 (DSM 5386) in einem Kohlenstoff- und Stickstoffquellen sowie anorganische Salze enthaltenden Medium in Gegenwart eines Adsorberharzes während der logarithmischen bis zur stationären Wachstumsphase aerob kultiviert,
(b) danach das Adsorberharz von der Kulturbrühe abtrennt und mit Wasser wäscht,
(c) die am Adsorberharz adsorbierten Ambruticine mit einem $C_{1-3}$-Alkanol auswäscht,
(d) das Eluat einengt und lyophilisiert,
(e) den Rückstand in einem $C_{1-2}$-Alkanol löst,
(f) die Lösung filtriert und über Sephadex chromatographiert und
(g) die ambruticinhaltigen Fraktionen einengt,

und fakultativ die so gewonnenen Ambruticine

(h) chromatographisch trennt

und/oder

(i) in ihre pharmazeutisch annehmbaren Salze überführt.

Erfindungsgemäß können die Ambruticine allein oder in Mischung mit geeigneten Trägern beispielsweise zum Behandeln von Pilzinfektionen bei Mensch und Tier und als Pflanzenschutzmittel verwendet werden.

Die folgenden Angaben erläutern die Erfindung.

Produktionsbedingungen

Produktionsstamm: Sorangium (Polyangium) cellulosum gehört zur Ordnung der Myxobacterales, Unterordnung Sorangineae, Familie Polyangiaceae. Der Produktionsstamm Sorangium cellulosum So ce 10 wurde im Mai 1978 an der GBF aus einer Bodenprobe von Isla Mujeres, Yucatan, Mexico, isoliert. Es

handelt sich um ein cellulosezersetzendes Bakterium. Der Stamm ist hinterlegt bei der Deutschen Sammlung von Mikroorganismen (DSM) unter der Nr. 5386.

Stammkultur und morphologische Beschreibung: Stammkulturen werden gehalten auf Hefeagar VY/2-Agar: 0.5 % Bäckerhefe nach Frischgewicht; 0.1 % $CaCl_2$; 1.5 % Agar; pH 7.2 oder auf Filterpapier über ST21-Agar (0.1 % $KNO_3$; 0.1 % $MgSO_4 \cdot 7H_2O$; 0.1 % $CaCl_2$; 0.1 % $K_2HPO_4$; 0.01 % $MnSO_4 \cdot 7H_2O$; 0.02 % $FeCl_3$; 0.002 % Hefeextrakt; Standard-Spurenelementlösung, z. B. nach G. Drews, Mikrobiologisches Praktikum, Berlin: Springer Verlag; 1 % Agar). Die Platten werden bei 30 °C bebrütet.

Auf Filterpapier über Mineralsalzagar oder auf Hefeagar (VY/2-Agar) bildet der Stamm große hell orange Schwarmkolonien mit vielen gelborange bis schwarzbraunen Fruchtkörpern. Letztere bestehen aus kleinen Sporangiolen von 15-20 µm Durchmesser, die in mehr oder weniger großen Haufen dicht gepackt liegen; die Haufen messen meist zwischen 50 und 150 µm Durchmesser. Die vegetativen Stäbchen haben die für Sorangium typische Form: relativ derbe, im Phasenkontrastmikroskop dunkle, zylindrische Stäbchen mit breit abgerundeten Enden, im Mittel 3-6 µm lang und um 1 µm dick. Nach längerer Adaptation an das Wachstum in Flüssigmedien wächst der Stamm in homogener Zellsuspension.

Biologische Charakterisierung des Stamms "So ce 10"

| | |
|---|---|
| Glucose-Abbau | positiv |
| Cellulose-Abbau | positiv |
| Stärke-Abbau | positiv |
| $NH_4^+$- als N-Quelle | positiv |
| $NO_3$- als N-Quelle | positiv |

Leistungen: Der Stamm So ce 10 produziert mehrere chemisch nah verwandte Verbindungen, die das Wachstum zahlreicher Pilze und Hefen hemmen. Gegen alle getesteten Bakterien waren die Wirkstoffe unwirksam. Die Wirkstoffe können sowohl aus den Zellen als auch aus dem Kulturüberstand isoliert werden.

Produktion der Wirkstoffe: Die Wirkstoffe werden während der logarithmischen bis hin zur stationären Wachstumsphase produziert.

Eine typische Fermentation verläuft folgendermaßen: Ein 100-l-Fermentor (Giovanola, Monthey, Schweiz) wird mit 63 l Medium (0.5 % Stärke, 0.5 % Sojabohnenmehl, 0.05 % $MgSO_4 \cdot 7H_2O$, 0.05 % $CaCl_2 \cdot 2H_2O$, 1 % (Volumenbasis) Adsorberharz XAD-1180 von Rohm und Haas, pH 7.4) gefüllt. Beimpft wird mit 7 l einer im gleichen Medium in Schüttelkolben angezogenen Vorkultur. Fermentiert wird bei 32 °C mit einer Rührgeschwindigkeit von 400 Upm und einer Belüftung von 0.2 $Nm^3$ pro Stunde. Schaumbildung wird durch Zugabe von 20 ml Silikon Antischaum verhindert. Nach vier Tagen ist die Fermentation abgeschlossen.

Die Wirkstoffe sind quantitativ an das Adsorberharz gebunden, welches durch Absieben des Fermentorinhalts über ein Sieb mit 0.2 mm Maschenweite gewonnen wird.

Wirkung

Hemmspektrum der Wirkstoffe im Plattendiffusionstest

Die Konzentration der in Methanol gelösten Wirkstoffe betrug 1 µg pro Testblättchen von 6 mm Durchmesser. Das Agarvolumen betrug 10 ml pro Petrischale von 9 cm Durchmesser. Das Wirkungsspektrum der beiden Hauptkomponenten ist identisch.

| Testorganismus | Hemmhofdurchmesser (mm) |
|---|---|
| Hefen: | |
| Candida albicans | 9 |
| Nematospora coryli | - |
| Pichia membranaefaciens | 10 |
| Debaryomyces hansenii | 11 |
| Rhodotorula glutinis | - |
| Torulopsis glabrata | - |
| Saccharomyces cerevisiae | - |
| Hansenula anomala | 10,6 |
| Schizosaccharomyces pombe | - |
| Nadsonia fulvescens | - |
| Hyphenpilze: | |
| Mucor hiemalis | 10,8 |
| Pythium debaryanum | 13,2 |
| Verticillum albo-atrum | 15 |
| Rhizopus sp. | 18 |
| Botrytis cinerea | 9,2 |

Minimale Hemmkonzentrationen

| Organismus | MHK (µg/ml) |
|---|---|
| Candida albicans | 1 |
| Debaryomyces hansenii | 0,5 |
| Mucor hiemalis | 0,1 |

Die Verbindungen zeigen damit in vitro gute Wirksamkeit sowohl gegen humanpathogene wie gegen phytopathogene Pilze.

Herstellung der Verbindungen VS-1, -3, -4, -5 in Gegenwart eines Adsorberharzes

Nach Beendigung der Fermentation wird das Adsorberharz (z. B. Amberlite XAD-1180) abgesiebt, mit Wasser gewaschen und in eine Glassäule gespült. In einem Zeitraum von 3 Stunden wird mit 3 Bettvolumen Methanol eluiert. Das Eluat wird im Vakuum bis zum Auftreten der Wasserphase eingeengt und anschließend lyophilisiert. Dabei erhält man als Rückstand 15 g eines dunkel gefärbten zähen Öls, das in 200 ml Methanol gelöst, filtriert und in 3 Portionen über Sephadex LH-20 chromatographiert wird (Säule 6 cm Durchmesser, 70 cm lang, Laufmittel Methanol, Detektion bei 254 nm und DC-Chromatographie). Die Komponenten VS-1, -3, -4, -5 eluieren weitgehend gemeinsam im letzten einer Gruppe breiter, sich überlagernder Peaks. Beim Eindampfen der entsprechenden Fraktion bleibt der Wirkstoff-Komplex als schwach gelb gefärbter Feststoff zurück (4.2 g). Seine Zusammensetzung hängt von den Fermentationsbedingungen ab; in der Regel stellt VS-3 die Hauptkomponente neben VS-4 und geringen Mengen VS-1 und VS-5 dar.

Trennung der Komponenten

0.13 g des Wirkstoff-Komplexes werden an HD-Sil-18-30-60 chromatographiert (Laufmittelgradient Methanol/Wasser von 85:15 nach 100:0, beide Laufmittelkomponenten enthalten 0.01 M Ammoniumacetat, pH 4.9). Die Komponenten VS-3 und -4 eluieren als getrennte Peaks, zuerst VS-4 dann VS-3. Aus den entsprechenden Fraktionen wird das Methanol im Vakuum abgedampft; der wässerige Rückstand wird lyophilisiert, wobei 34 mg VS-4 bzw. 38 mg VS-3 als farblose amorphe Feststoffe erhalten werden. Aus Vor- und Nachlauf können durch präparative Schichtchromatographie (PSC-Fertigplatten-Kieselgel 60 F254 Schicht 1 mm, Merck, Laufmittel Methanol/Eisessig 99:1, Detektion 254 nm) mit einer Ausbeute von 3 mg

VS-5 bzw. 6 mg VS-1 erhalten werden.

Die erfindungsgemäßen Ambruticine besitzen die folgende Struktur:

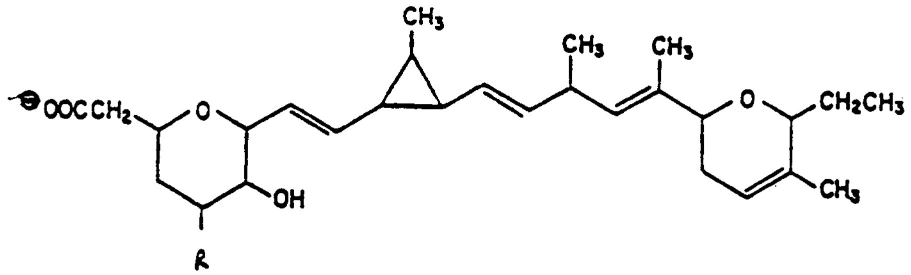

I

Ambruticin

VS-1 R = $N(CH_3)_3^+$

VS-3 R = $NH(CH_3)_2^+$

VS-4 R = $NH_2CH_3^+$

VS-5 R = $NH_3^+$

Bevorzugt liegen die Ambruticine in folgender Konfiguration vor:

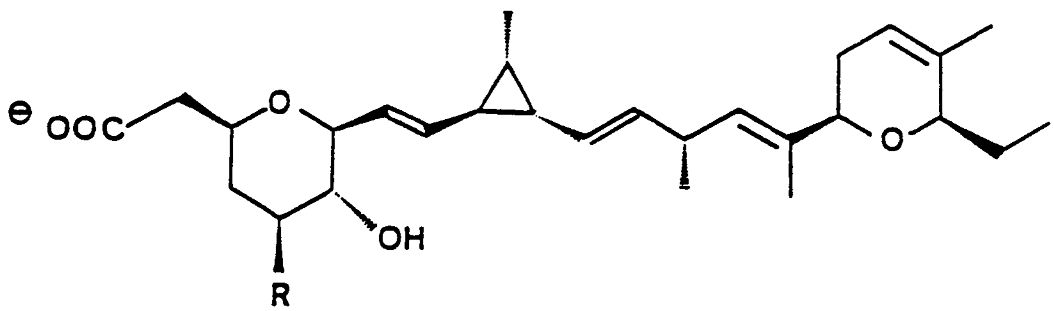

II

jedoch fallen alle durch Einwirkung von Licht oder Wärme erzeugbaren Konfigurations-Isomere ebenfalls unter diese Erfindung.

<u>Chemische Charakterisierung von Ambruticin VS-1</u>

Summenformel: $C_{31}H_{49}NO_5$ MG:515
Farblose amorphe Substanz
UV (MeOH) Endabsorption
MS (FAB, Matrix m-Nitrobenzylalkohol, pos. Ionen) = 516 $(M+H)^+$ Hochauflösung
ber.: 516.3676
gef.: 516.3678
$^1$H-NMR : Tab. 1
$^{13}$C-NMR: Tab. 2
DC (DC-Alufolie 60 $F_{254}$, Merck; Laufmittel: Methanol/Eisessig 99:1) $R_f$ = 0.15
Detektion durch Ansprühen mit Vanillin/Schwefelsäure-Reagens und Erhitzen auf 120 °C, blau-grauer Fleck)

<u>Chemische Charakterisierung von Ambruticin VS-3</u>

Summenformel: $C_{30}H_{47}NO_5$ MG:501
Farblose amorphe Substanz
$[\alpha]_D^{22}$ = 90.5° (MeOH; c = 2.5)
UV (MeOH) Endabsorption
MS (FAB, Matrix m-Nitrobenzylalkohol, neg. Ionen) = 500 $(M-H)^-$ Hochauflösung

ber.: 500.3364
gef.: 500.3378
$^1$H-NMR : Tab. 1
$^{13}$C-NMR: Tab. 2
DC (DC-Alufolie 60 $F_{254}$, Merck; Laufmittel: Methanol/Eisessig 99:1) $R_f$ = 0.32
Detektion durch Ansprühen mit Vanillin/Schwefelsäure-Reagens und Erhitzen auf 120 °C, blau-grauer Fleck)

Chemische Charakterisierung von Ambruticin VS-4

Summenformel: $C_{29}H_{45}NO_5$ MG:487
Farblose amorphe Substanz
$[\alpha]_D^{25}$ = 101.4° (MeOH; c = 1.5)
UV (MeOH) Endabsorption
MS (FAB, Matrix m-Nitrobenzylalkohol, neg. Ionen) = 486 $(M\text{-}H)^-$ Hochauflösung
ber.: 486.3208
gef.: 486.3239
$^1$H-NMR : Tab. 1
$^{13}$C-NMR: Tab. 2
DC (DG-Alufolie 60 $F_{254}$, Merck; Laufmittel: Methanol/Eisessig 99:1) $R_f$ = 0.51
Detektion durch Ansprühen mit Vanillin/Schwefelsäure-Reagens und Erhitzen auf 120 °C, blau-grauer Fleck)

Chemische Charakterisierung von Ambruticin VS-5

Summenformel: $C_{28}H_{43}NO_5$ MG:473
Farblose amorphe Substanz
UV (MeOH) Endabsorption
MS (FAB, Matrix m-Nitrobenzylalkohol, neg. Ionen) = 472 $(M\text{-}H)^-$ Hochauflösung
ber.: 472.3052
gef.: 472.3051
$^1$H-NMR : Tab. 1
$^{13}$C-NMR: Tab. 2
DC (DC-Alufolie 60 $F_{254}$, Merck; Laufmittel: Methanol/Eisessig 99:1) $R_f$ = 0.70
Detektion durch Ansprühen mit Vanillin/Schwefelsäure-Reagens und Erhitzen auf 120 °C, blau-grauer Fleck)

Tab. 1 $^1$H-NMR Spektroskopische Daten der Ambruticine VS in [$D_4$]Methanol

| H-Atom | VS-1 | VS-3 | VS-4 | VS-5 | $J_{H,H}$ (Hz) |
|---|---|---|---|---|---|
| | δ (ppm) Multiplizität | | | | |
| 2a | 2.36 dd | 2.37 dd | 2.37 m | 2.30 m | 2a,2b - 15.0 |
| 2b | 2.57 dd | 2.58 dd | 2.55 dbr | 2.55 dd | 2a,3 - 6.5 |
| 3 | 3.85 m | 3.93 m | 3.94 m | 3.93 m | 2b, 3 - 6.9 |
| 4a | 1.55 m | 1.55 ddd | 1.45 m | 1.55 m | 3, 4a - 7.0 |
| 4b | 2.15 m | 2.22 dbr | 2.30 dbr | 2.21 dbr | 4a,4b - 14.2 |
| 5 | 3.60 m | 3.36 m | 3.16 m | 3.15 m | 4a, 5 - 6.0 |
| 6 | 3.60 m | 3.40 m | 3.28 dd | 3.15 m | 6 , 7 - ca.8 |
| 7 | 3.60 m | 3.66 m | 3.63 m | 3.63 m | 7 , 8 - 6.0 |

| H-Atom | VS-1, VS-3, VS-4, VS-5 δ (ppm) Multiplizität | $J_{H,H}$ (Hz) |
|---|---|---|
| 8 | 5.55 m | 9, 10 - 7.9 |
| 9 | 5.47 m | 12, 13 - 8.8 |
| 10 | 1.19 m | 13, 14 - 15.2 |
| 11 | 1.10 m | 13, 15 - 1.2 |
| 12 | 1.54 m | 14, 15 - 6.5 |
| 13 | 5.21 ddd | 15, 16 - 9.0 |
| 14 | 5.50 m | 15,15-Me - 7.0 |
| 15 | 3.37 ddq | 16,17-Me - 1.2 |
| 16 | 5.28 ddq | 16, 18 - 1.3 |
| 18 | 3.87 ddd | 18,19a - 3.0 |
| 19a | 1.91 m | 18,19b - 10.5 |
| 19b | 2.13 m | 19a,20 - 6.2 |
| 20 | 5.62 m | 19,21-Me - 1.2 |
| 22 | 4.13 m | 20,21-Me - 1.2 |
| 23a | 1.79 m | 23, 24 - 7.4 |
| 23b | 1.58 m | |
| 24 | 0.94 t | |
| 11-Me | 1.10 sbr | |
| 15-Me | 1.09 d | |
| 17-Me | 1.69 d | |
| 21-Me | 1.64 tq | |

| H-Atom | VS-1 | VS-3 | VS-4 |
|---|---|---|---|
| N-Me | 3.25 s | 2.83 s | 2.72 s |

Folgende NMR-Geräte wurden verwendet: Bruker/Karlsruhe WM-400 und AM-300.

Tab. 2 $^{13}C$-Chemische Verschiebungen der Ambruticine VS in $[D_4]$Methanol

| | VS-1 | VS-3 | VS-4 |
|---|---|---|---|
| C-Atom | | δ (ppm) | |
| 1 | 178.7* | 178.0* | - * |
| 2 | 44.9 | 44.8 | 44.8 |
| 3 | 74.7 | 74.7 | 74.5 |
| 4 | 33.0 | 30.8 | 33.6 |
| 5 | 76.4 | 68.6 | 62.5 |
| N-Me | 53.7 | 40.3 | 31.6 |
| 6 | 72.0 | 70.4 | 72.4 |
| 7 | 82.7 | 82.4 | 82.3 |
| 8 | | 125.4 | |
| 9 | | 138.4 | |
| 10 | | 31.6 | |
| 11 | | 22.4 | |
| 12 | | 29.8 | |
| 13 | | 126.8 | |
| 14 | | 136.5 | |
| 15 | | 36.2 | |
| 16 | | 130.7 | |
| 17 | | 136.3 | |
| 18 | | 79.4 | |
| 19 | | 31.2 | |
| 20 | | 122.0 | |
| 21 | | 136.2 | |
| 22 | | 79.5 | |
| 23 | | 26.6 | |
| 24 | | 8.7 | |
| 25 | | 13.3 | |
| 26 | | 21.7 | |
| 27 | | 12.7 | |
| 28 | | 19.0 | |

* wegen Austauschs breit bzw. nicht beobachtet

Verzeichnis der verwendeten Abkürzungen

| | |
|---|---|
| Sephadex LH-20 | (Fa. Pharmazia) |
| HD-Sil-18-30-60 | (Fa. Kronwald) |
| Amberlite XAD-1180 | (Fa. Rohm und Haas) |
| PSC Fertigplatten Kieselgel 60 $F_{254}$ | (Fa. Merck). |

## Patentansprüche

1. Stickstoffhaltige Ambruticine der allgemeinen Formel

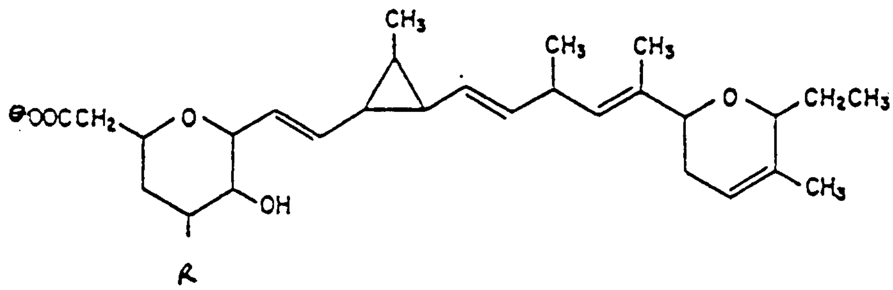

I

worin R $N(CH_3)_3^+$, $NH(CH_3)_2^+$, $NH_2CH_3^+$ oder $NH_3^+$ bedeutet, und deren pharmazeutisch und agrikulturchemisch annehmbare Salze.

2. Stickstoffhaltige Amoruticine nach Anspruch 1 mit der in Formel II angegebenen Konfiguration

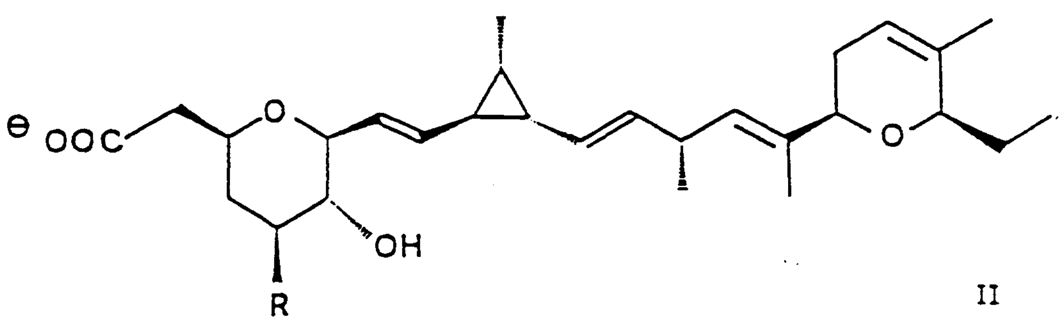

II

und deren pharmazeutisch und agrikulturchemisch annehmbare Salze.

3. Verfahren zum Herstellen stickstoffhaltiger Ambruticine und ihrer pharmazeutisch annehmbaren Salze, dadurch *gekennzeichnet*, daß man
   (a) Sorangium (Polyangium) cellulosum So ce 10 (DSM 5386) in einem Kohlenstoff- und Stickstoffquellen sowie anorganische Salze enthaltenden Medium in Gegenwart eines Adsorberharzes während der logarithmischen bis zur stationären Wachstumsphase aerob kultiviert,
   (b) danach das Adsorberharz von der Kulturbrühe abtrennt und mit Wasser wäscht,
   (c) die am Adsorberharz adsorbierten Ambruticine mit einem $C_{1-3}$-Alkanol auswäscht,
   (d) das Eluat einengt und lyophilisiert,
   (e) den Rückstand in einem $C_{1-3}$-Alkanol löst,
   (f) die Lösung filtriert und über Sephadex chromatographiert und
   (g) die ambruticinhaltigen Fraktionen einengt,

   und fakultativ die so gewonnen Ambruticine
   (h) chromatographisch trennt und/oder
   (i) in ihre pharmazeutisch annehmbaren Salze überführt.

4. Verfahren nach Anspruch 2, dadurch *gekennzeichnet*, daß man die chromatographische Trennung gemäß (h) durchführt, indem man
   ($h_1$) die Fraktion der Ambruticine an HD-Sil-18-30-60 mit Methanol/Wasser als Laufmittel chromatographiert,
   ($h_2$) die die Ambruticine VS-4 (R = $NH_2CH_3$)und VS-3 (R = $NH(CH_3)_2$) enthaltenden Fraktionen vom Methanol befreit und
   ($h_3$) die wässerigen Rückstände jeweils lyophilisiert und/oder
   ($h_{2'}$) aus Vor- und Nachlauf durch präparative Schichtchroma tographie auf Kieselgel die Ambruticine VS-5 (R = $NH_3$) und
   VS-1 (R = $N(CH_3)_3$) isoliert.

5. Stickstoffhaltige Ambruticine oder ihre pharmazeutisch annehmbare Salze nach einem der Ansprüche 1 bis 3, allein oder in Mischung mit geeigneten Trägern, zum Behandeln von Pilzinfektionen bei Mensch und Tier.

6. Verwendung der stickstoffhaltigen Ambruticine oder ihrer pharmazeutisch annehmbaren Salze nach einem der Ansprüche 1 bis 3, allein oder in Mischung mit geeigneten Trägern, als Pflanzenschutzmittel.

**Claims**

1. Nitrogen-containing ambruticins of the general formula

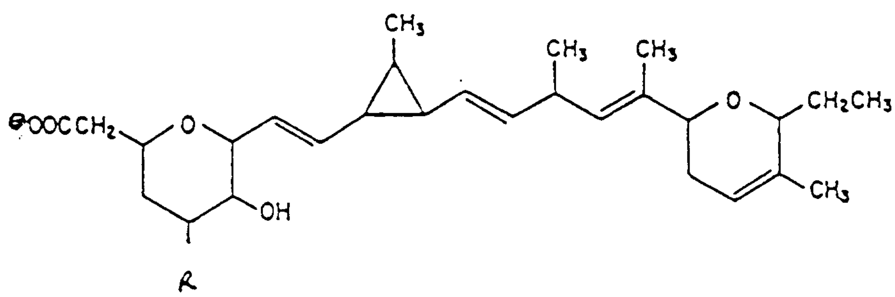

I

in which R denotes $N(CH_3)_3^+$, $NH(CH_3)_2^+$, $NH_2CH_3^+$ or $NH3^+$, and their pharmaceutically and agrochemically acceptable salts.

2. Nitrogen-containing ambruticins according to claim 1 that have the configuration given in formula II

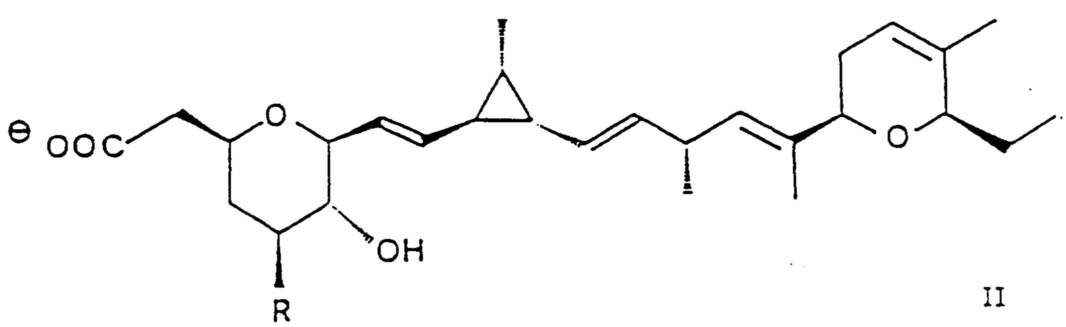

II

and their pharmaceutically and agrochemically acceptable salts.

3. Process for preparing nitrogen-containing ambruticins and their pharmaceutically acceptable salts, characterized in that
   (a) Sorangium (Polyangium) cellulosum So ce 10 (DSM 5386) is cultured aerobically during the logarithmic growth phase to the stationary growth phase in a medium that contains carbon and nitrogen sources as well as inorganic salts, in the presence of an adsorber resin,
   (b) the adsorber resin is then removed from the culture liquid and washed with water,
   (c) the ambruticins that are adsorbed on the adsorber resin are removed by washing with a $C_{1-3}$-alkanol,

(d) the eluate is concentrated and lyophilized,
(e) the residue is dissolved in a $C_{1-3}$-alkanol,
(f) the solution is filtered and chromatographed over Sephadex and
(g) the ambruticin -containing fractions are concentrated,

and, if desired, the ambruticins so obtained

(h) are separated by chromatography and/or
(i) converted into their pharmaceutically acceptable salts.

**4.** Process according to claim 2, characterized in that the chromatographic separation according to (h) is carried out by

($h_1$) chromatographing the ambruticin fraction on HD-Sil-18-30-60 using methanol/water as the eluant,
($h_2$) freeing the fractions that contain the ambruticins VS-4 (R = $NH_2CH_3$) and VS-3 (R = $NH(CH_3)_2$) from methanol, and
($h_3$) in each case lyophilizing the aqueous residues and/or
($h_{2'}$) isolating the ambruticins VS-5 (R = $NH_3$) and VS-1 (R = $N(CH_3)_3$) from the preliminary and final fractions by preparative layer chromatography on silica gel.

**5.** Nitrogen-containing ambruticins or their pharmaceutically acceptable salts according to any one of claims 1 to 3, on their own or as a mixture with suitable carriers, for treating fungal infections in humans and animals.

**6.** The use of nitrogen-containing ambruticins or their pharmaceutically acceptable salts according to any one of claims 1 to 3, on their own or as a mixture with suitable carriers, as crop protection agents.

**Revendications**

**1.** Ambruticines azotées, répondant à la formule générale :

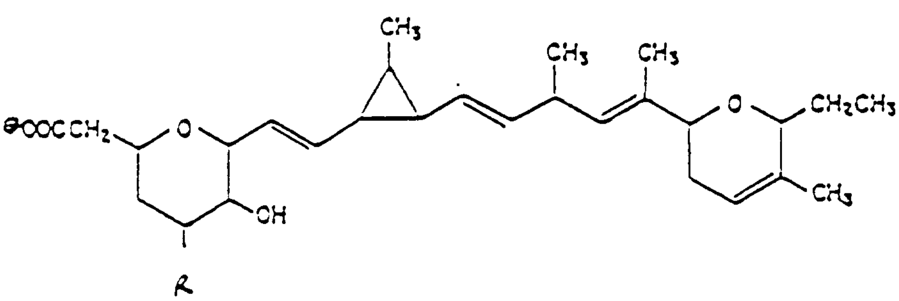

I

dans laquelle R désigne $N(CH_3)_3^+$, $NH(CH_3)_2^+$, $NH_2CH_3^+$ ou $NH_3^+$, et leurs sels acceptables du point de vue pharmaceutique et agrochimique.

**2.** Ambruticines azotées selon la revendication 1, ayant la configuration indiquée dans la formule II ;

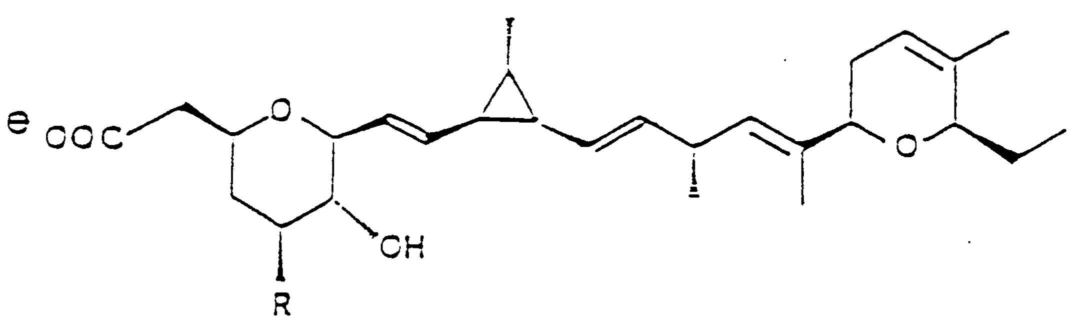

II

et leurs sels acceptables du point de vue pharmaceutique et agrochimique.

**3.** Procédé de préparation d'ambruticines azotées et de leurs sels pharmaceutiquement acceptables, caractérisé en ce que :

(a) on cultive, en aérobie, Sorangium (Polyangium) cellulosum So ce 10 (DSM 5386), dans un milieu contenant des sources de carbone et d'azote ainsi que des sels minéraux, en présence d'une résine adsorbante pendant la phase de croissance logarithmique jusqu'à la phase de croissance stationnaire,

(b) on sépare ensuite la résine adsorbante du bouillon de culture et on la lave à l'eau,

(c) on extrait par lavage les ambruticines adsorbées sur la résine adsorbante avec un alcanol en $C_1$ à $C_3$,

(d) on concentre l'éluat et on le lyophilise,

(e) on dissout le résidu dans un alcanol en $C_1$ à $C_3$,

(f) on filtre la solution et on la chromatographie sur Sephadex, et

(g) on concentre les fractions contenant l'ambruticine, et facultativement,

(h) on sépare par chromatographie les ambruticines ainsi obtenues, et/ou

(i) on les transforme en leurs sels pharmaceutiquement acceptables.

**4.** Procédé selon la revendication 2, caractérisé en ce qu'on effectue une séparation chromatographique selon (h),

($h_1$) en chromatographiant la fraction des ambruticines sur HD-Sil-18-30-60 avec du méthanol/eau comme éluant,

($h_2$) en éliminant le méthanol des fractions contenant les ambruticines VS-4($R = NH_2CH_3$) et VS-3-($R = NH(CH_3)_2$), et

($h_3$) en lyophilisant, chaque fois, les résidus aqueux, et/ou

($h_{2'}$) en isolant des têtes et des queues, par chromatographie préparative sur couche de gel de silice, les ambruticines VS-5 ($R = NH_3$) et VS-1 ($R = N(CH_3)_3$).

**5.** Ambruticines azotées ou leurs sels pharmaceutiquement acceptables, selon l'une des revendications 1 à 3, seules ou en mélange, avec des supports appropriés, pour le traitement des mycoses chez l'homme et chez les animaux.

**6.** Utilisation des ambruticines azotées ou de leurs sels pharmaceutiquement acceptables selon l'une des revendications 1 à 3, seules ou en mélange, avec des supports appropriés, comme produits phytosanitaires.